# EUROPEAN PATENT SPECIFICATION

(11) **EP 0 973 889 B1**
(45) Date of publication and mention of the grant of the patent: **26.10.2005**
(21) Application number: 98905192.5
(22) Date of filing: 27.02.1998
(51) Int. Cl.: C12N 15/12, C07K 14/705, C12N 5/10, C12Q 1/68, G01N 33/68

(54) **HUMAN CALCIUM CHANNELS ALFA1i SUBUNITS AND RELATED PROBES, CELL LINES AND METHODS**
MENSCHLICHE KALZIUMKANÄLE ALFA1i UNTEREINHEITEN UND VERWANDTE PROBEN, ZELLLINIEN UND VERAHREN
SOUS-UNITES ALFA-1i DE CANAUX A CALCIUM HUMAINES ET SONDES, LIGNEES CELLULAIRES ET PROCEDES ASSOCIES

(30) Priority: 28.02.1997 US 39204 P; 25.02.1998 US 30482
(43) Date of publication of application: 26.01.2000
(73) Proprietor: Neuromed Technologies Inc., Vancouver, British Columbia V6T 1Z3 (CA)
(72) Inventor: SNUTCH, Terry, P., Vancouver, British Columbia V6T 1Z3 (CA); BAILLIE, David, L., Vancouver, BC V5Z 3Z3 (CA)
(74) Representative: Lucas, Brian Ronald
(86) International application number: PCT/CA1998/000173
(87) International publication number: WO 1998/038301

(56) References cited:
- WO-A-95/04144
- WO-A1-99/29847
- WO-A2-01/02561
- TROFATTER JA ET AL: "An expression-independent catalog of genes from human chromosome 22." GENOME RES, OCT 1995, 5 (3) P214-24, UNITED STATES, XP002069420 & EMBL database Accession number H55544 08-12-95 Trofatter j.a. et al.
- MCRORY J.E.: 'Molecular and functional characterization of a family of rat brain T-type calcium channels' THE JOURNAL OF BIOLOGICAL CHEMISTRY vol. 276, no. 6, 09 February 2001, pages 3999 - 4011

## Description

### TECHNICAL FIELD

The present invention relates to novel human calcium channel compositions, and to the expression of these compositions in cell lines for use in evaluating calcium channel function.

### BACKGROUND OF THE INVENTION

The rapid entry of calcium into cells is mediated by a class of proteins called voltage-gated calcium channels. Calcium channels are a heterogeneous class of molecules that respond to depolarization by opening a calcium-selective pore through the plasma membrane. The entry of calcium into cells mediates a wide variety of cellular and physiological responses including excitation-contraction coupling, hormone secretion and gene expression. In neurons, calcium entry directly affects membrane potential and contributes to electrical properties such as excitability, repetitive firing patterns and pacemaker activity. Miller, R.J. (1987) Multiple calcium channels and neuronal function. Science 235:46-52. Calcium entry further affects neuronal functions by directly regulating calcium-dependent ion channels and modulating the activity of calcium-dependent enzymes such as protein kinase C and calmodulin-dependent protein kinase II. An increase in calcium concentration at the presynaptic nerve terminal triggers the release of neurotransmitter. Calcium entry also plays a role in neurite outgrowth and growth cone migration in developing neurons and has been implicated in long-term changes in neuronal activity. In addition to the variety of normal physiological functions mediated by calcium channels, they are also implicated in a number of human disorders. Recently, mutations identified in human and mouse calcium channel genes have been found to account for several disorders including, familial hemiplegic migraine, episodic ataxia type 2, cerebellar ataxia, absence epilepsy and seizures. Fletcher, et al. (1996) Absence epilepsy in tottering mutant mice is associated with calcium channel defects. Cell 87:607-617: Burgess, et al. (1997) Mutation of the Ca2+ channel β subunit gene Cchb4 is associated with ataxia and seizures in the lethargic (1h) mouse. Cell 88:385-392; Ophoff, et al. (1996) Familial hemiplegic migraine and episodic ataxia type-2 are caused by mutations in the Ca2+ channel gene CACNL1A4. cell 87:543-552; Zhuchenko, O. et al. (1997) Autosomal dominat cerebellar ataxia (SCA6) associated with the small polyglutamine expansions in the α1A-voltage-dependent calcium channel. Nature Genetics 15:62-69.

The clinical treatment of some disorders has been aided by the development of therapeutic calcium channel antagonists. Janis, et al. (1991) In Calcium Channels: Their Properties, Functions. Regulation and Clinical Relevance. CRC Press, London.

Native calcium channels have been classified by their electrophysiological and pharmacological properties as T, L, N, P and Q types (for reviews see McCleskey, et al. (1991) Functional properties of voltage-dependent calcium channels. Curr. Topics Membr. 39: 295-326, and Dunlap, et al. (1995) Exocytotic Ca²⁺ channels in mammalian central neurons. Trends Neurosci. 18:89-98.). T-type (or low voltage-activated) channels describe a broad class of molecules that transiently activate at negative potentials and are highly sensitive to changes in resting potential. The L, N, P and Q-type channels activate at more positive potentials and display diverse kinetics and voltage-dependent properties. There is some overlap in biophysical properties of the high voltage-activated channels, consequently pharmacological profiles are useful to further distinguish them. L-type channels are sensitive to dihydropyridine (DHP) agonists and antagonists, N-type channels are blocked by the *Conus geographus* peptide toxin, ω-conotoxin GVIA, and P-type channels are blocked by the peptide ω-agatoxin IVA from the venom of the funnel web spider, *Agelenopsis aperta.* A fourth type of high voltage-activated Ca channel (Q-type) has been described, although whether the Q- and P-type channels are distinct molecular entities is controversial (Sather et al. (1993) Distinctive biophysical and pharmacological properties of class A (B1) calcium channel a 1 subunits. Neuron 11: 291-303; Stea, et al. (1994) Localization and functional properties of a rat brain α1A calcium channel reflect similarities to neuronal Q- and P-type channels. Proc Natl Acad Sci (USA) 91: 10576-10580.). Several types of calcium conductances do not fall neatly into any of the above categories and there is variability of properties even within a category suggesting that additional calcium channels subtypes remain to be classified.

Biochemical analyses show that neuronal calcium channels are heterooligomeric complexes consisting of three distinct subunits (α₁, α₂δ and β)( reveiwed by De Waard, et al. (1997) In Ion Channels, Volume 4, edited by Narahashi, T. Plenum Press, New York). The α₁ subunit is the major pore-forming subunit and contains the voltage sensor and binding sites for calcium channel antagonists. The mainly extracellular α₂ is disulphide-linked to the transmembrane δ subunit and both are derived from the same gene and are proteolytically cleaved *in vivo.* The β subunit is a non-glycosylated, hydrophilic protein with a high affinity of binding to a cytoplasmic region of the α₁ subunit. A fourth subunit, γ, is unique to L-type Ca channels expressed in skeletal muscle T-tubules. The isolation and characterization of γ-subunit-encoding cDNAs is described in US Patent No. 5,386,025 which is incorporated herein by reference.

Molecular cloning has revealed the cDNA and corresponding amino acid sequences of six different types of α₁ subunits (α_{1A}, α_{1B}, α_{1C}, α_{1D}, α_{1E} and α_{1S}) and four types of β subunits (β₁, β₂, β₃ and β₄)(reviewed in Stea, A., Soong, T.W. and Snutch, T.P. (1994) Voltage-gated calcium channels. PCT Patent Publication WO 95/04144, which is incorporated herein by reference, discloses the sequence and expression of α_{1E} calcium channel subunits. In Handbook of Receptors and Channels. Edited by R.A. North, CRC Press.).

The different classes of α1 and β subunits have been identified in different animals including, rat, rabbit and human and share a significant degree of amino acid conservation across species (for examples see: Castellano, et al. (1993) Cloning and expression of a third calcium channel β subunit. J. Biol. Chem. 268: 3450-3455; Castellano, et al. (1993) Cloning and expression of a neuronal calcium channel β subunit. J. Biol. Chem. 268: 12359-12366; Dubel, et al. (1992). Molecular cloning of the α₁ subunit of an ω-conotoxin-sensitive calcium channel. Proc. Natl. Acad. Sci. (USA) 89: 5058-5062; Fujita, et al.. (1993) Primary structure and functional expression of the ω-conotoxin-sensitive N-type calcium channel from rabbit brain. Neuron 10: 585-598; Mikami, et al., (1989). Primary structure and functional expression of the cardiac dihydropyridine-sensitive calcium channel. Nature 340: 230-233; Mori, et al. (1991) Primary structure and functional expression from complementary DNA of a brain calcium channel. Nature 350: 398-402; Perez-Reyes, et al. (1992). Cloning and expression of a cardiac/brain β subunit of the L-type calcium channel. J. Biol. Chem. 267: 1792-1797; Pragnell, et al. (1991). Cloning and tissue-specific expression of the brain calcium channel β-subunit. FEBS Lett. 291: 253-258; Snutch, et al. (1991) Distinct calcium channels are generated by alternative splicing and are differentially expressed in the mammalian CNS. Neuron 7: 45-57; Soong, et al. (1993) Structure and functional expression of a member of the low voltage-activated calcium channel family. Science 260: 1133-1136; Tomlinson, et al. (1993) Functional properties of a neuronal class C L-type channel. Neuropharmacology 32: 1117-1126; Williams, et al. (1992) Structure and functional expression of α1, α2, and β subunits of a novel human neuronal calcium channel subtype. Neuron 8: 71-84; Williams, et al. (1992) Structure and functional expression of an ω-conotoxin-sensitive human N-type calcium channel. Science 257: 389-395.

In some expression systems the α₁ subunits alone can form functional calcium channels although their electrophysiological and pharmacological properties can be differentially modulated by coexpression with any of the four β subunits. Until recently, the reported modulatory affects of β subunit coexpression were to mainly alter kinetic and voltage-dependent properties. More recently it has been shown that β subunits also play crucial roles in modulating channel activity by protein kinase A, protein kinase C and direct G-protein interaction. (Bourinet, et al. (1994) Voltage-dependent facilitation of a neuronal α1C L-type calcium channel. EMBO J. 13: 5032-5039; Stea, et al. (1995) Determinants of PKC-dependent modulation of a family of neuronal calcium channels. Neuron 15:929-940; Bourinet, et al. (1996) Determinants of the G-protein-dependent opioid modulation of neuronal calcium channels. Proc. Natl. Acad. Sci. (USA) 93: 1486-1491.)

The electrophysiological and pharmacological properties of the calcium channels cloned to date can be summarized as shown in Table 1. While the cloned α₁ subunits identified to date correspond to several of the calcium channels found in cells, they do not account for all types of calcium conductances described in native cells. For example, they do not account for all types of calcium conductances described in native cells. For example, they do not account for the various properties desecibed for the heterogenous family described as T-type calcium channels. Furthermore, they do not account for novel calcium channels described in cerebellar granule cells or other types of cells. (Forti, et al (1993) Functional diversity of L-type calcium channels in rat cerebellar neurons. Neuron 10: 437-450; Tottene, et al. (1996). Functional diversity of P-type and R-type calcium channels in rat cerebellar neurons. J. Neurosci. 16: 6353-6363).

Because of the importance of calcium channels in cellular metabolism and human disease, it would be desirable to identify the remaining classes of α₁ subunits, and to develop expression systems for these subunits which would permit the study and characterization of these calcium channels, including the study of pharmacological modulators of calcium channel function. Thus, it is an object of the present invention to provide heretofor undisclosed calcium channels having novel α₁ subunits, including cell lines expressing these new calcium channels. It is a further object of the present invention to provide a method for testing these novel calcium channels using such cell lines.

### SUMMARY OF THE INVENTION

The present invention provides partial sequences for a novel mammalian (human and rat sequences identified) calcium channel subunit which we have labeled as the α₁ᵢ subunit. This knowledge of the sequence of this calcium channels permits the localization and recovery of the complete sequence from human cells, and the development of cell lines which express the novel calcium channels of the invention. These cells may be used for identifying compounds capable of acting as agonists or antagonists to the calcium channels.

### BRIEF DESCRIPTION OF THE DRAWINGS

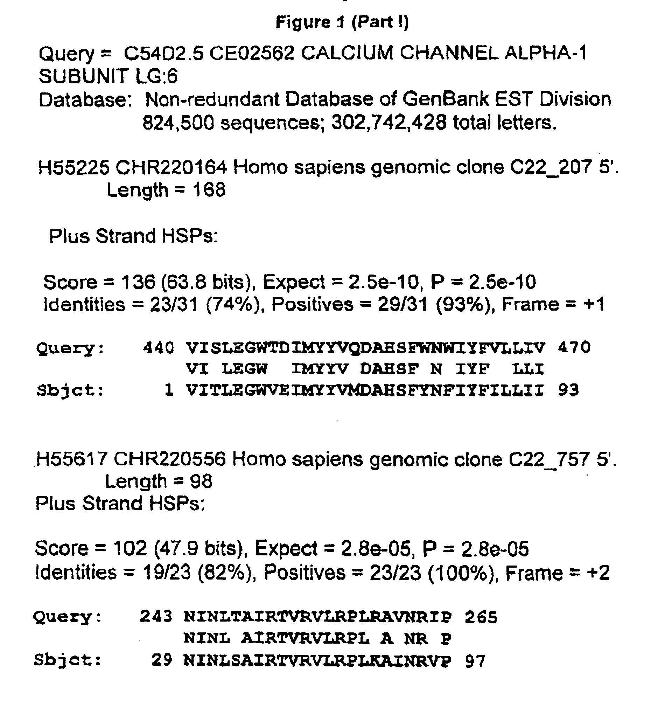

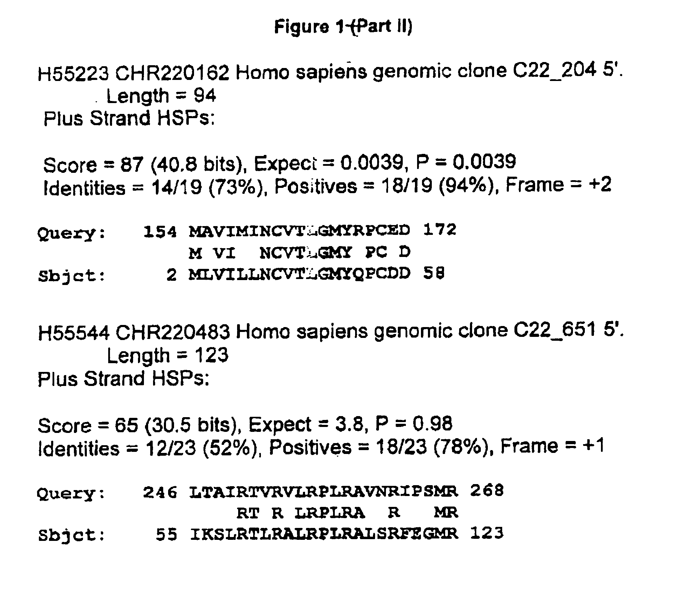

Fig. 1 shows aligned amino acid sequences for the *C. elegans* C54D2.5 α₁ calcium channel subunit and initially identified portions of the calcium channel subunits of the invention.

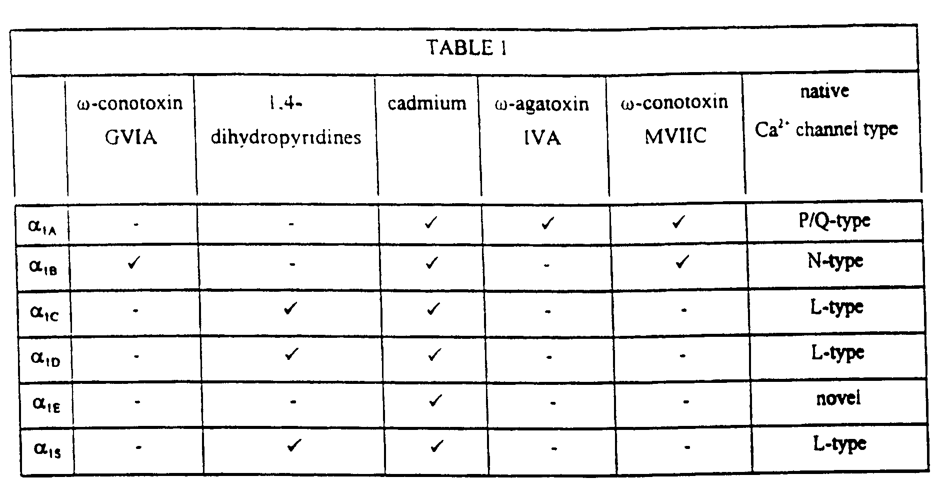

### DESCRIPTION OF THE INVENTION

The present invention includes the following aspects for which protection is sought:
(a) novel human calcium channel subunits and DNA fragments encoding such subunits. It will be appreciated that polymorphic variations may be made or may exist in the DNA of some individuals leading to minor deviations in the DNA or amino acids sequences from those shown which do not lead to any substantial alteration in the function of the calcium channel. Such variations, including variations which lead to substitutions of amino acids having similar properties are considered to be within the scope of the present invention.
(b) polynucleotide sequences useful as probes in screening human cDNA libraries for genes encoding these novel calcium channel subunits. These probes can also be used in histological assay to determine the tissue distribution of the novel calcium channel subunits.
(c) eukaryotic cell lines expressing the novel calcium channel subunits. These cell lines can be used to evaluate compounds as pharmacological modifiers of the function of the novel calcium channel subunits.
(d) a method for evaluating compounds as pharmacological modifiers of the function of the novel calcium channel subunits using the cell lines expressing those subunits alone or in combination with other calcium channel subunits.

Further, since defects in the novel calcium channel subunits may be associated with a human genetic disease including, but not limited to; epilepsy, migraine, ataxia, schizophrenia, hypertension, arrhythmia, angina, depression, small lung carcinoma, Lambert-Eaton syndrome, characterization of such associations and ultimately diagnosis of associated diseases can be carried out with probes which bind to the wild-type or defective forms of the novel calcium channels.

In accordance with the present invention, we have identified human DNA sequences which code for novel calcium channel α₁ subunits. These subunits are believed to represent a new types of α₁ subunits of human voltage-dependent calcium channels which has been designated as type α₁ᵢ.

The novel α₁ subunits of the invention were identified by screening the *C*. *elegans* genomic DNA sequence data base for sequences homologous to previously identified mammalian calcium channel α₁ subunits. Specifically, the following twelve mammalian α₁ subunit sequences were used to screen the *C. elegans* genomic data bank:

This search identified four distinct *C. elegans* cosmids that contain open reading frames (coding regions) that exhibit homology to mammalian calcium channel α₁ subunits:
cosmid and reading frame T02C5.5
cosmid and reading frame C48A7.1
cosmid and reading frame C54D2.5
cosmid and reading frame C27F2.3
Examination of the four *C. elegans* cosmid sequences by phylogeny analysis shows that two of these, T02C5.5 and C48A7.1, correspond closely with previously identified mammalian α₁ subunits. T02C5.5 appears to be an ancestral member related to the mammalian α_{1A}, α_{1B} and α_{1E} subunits. C48A7.1 appears to be an ancestral member related to the mammalian L-type channels encoded by α_{1C}, α_{1D} and α_{1S}. In contrast, the *C. elegans* cosmids C54D2.5 and C27F2.3 identify novel types of calcium channel α₁ subunits distinct from the other mammalian subtypes.

Mammalian counterparts of the *C. elegans* calcium channel α₁ subunit encoded by C54D2.5 were identified by screening of the GenBank expressed sequence tag (EST) data bank. This analysis identified a total of 13 mammalian sequences that exhibit some degree of DNA sequence and amino acid identity to C54D2.5, of which 8 are human sequences. (Table 2) Three of these sequences appear unlikely to encode novel calcium channel subunits because they either exhibit a significant degree of homology to previously identified mammalian α₁ subunits (clones H06096 and H14053) or exhibit homology in a region not considered to be diagnostic of calcium channel α₁ subunits specifically as opposed to other types of ion channel molecules in general (clone D20469). Four of the five remaining sequences (H55225, H55617, H55223, and H55544), however, are believed to encode a previously unidentified calcium channel α₁ subunits because the degree of amino acid identity closely matches that of known calcium channel subunits in conserved regions but is sufficiently different to indicate that they do not encode previously identified mammalian calcium channel α₁ subunits, α_{1A}, α_{1B}, α_{1C}, α_{1D}, α_{1E}, or α_{1S.} The expected amino acid sequence closely matches but is not identical to amino acid sequences in these known calcium channel subunits. The aligned amino acids sequences are shown in Fig 1.

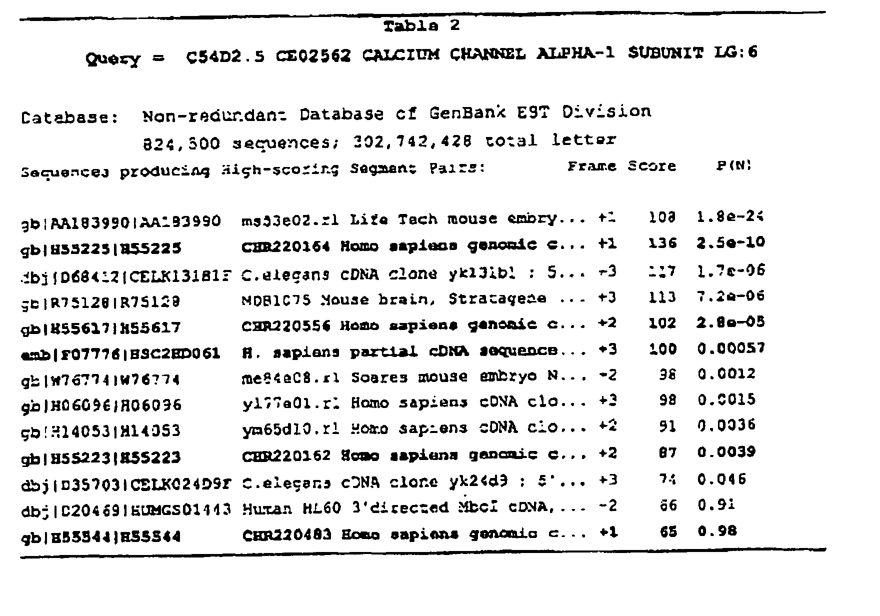

The four sequences (H55225, H55617, H55223, and H55544) are found on human chromosome 22, and are now believed to all be part of the same gene encoding the novel human calcium channel subunit α₁ᵢ.

The sequences of the five selected sequences and the references from which they are taken are given as follows:

A search of the Sanger Genome Sequencing Center (Cambridge, U.K.) and the Washington University Genome Sequencing Center (St. Louis. MO) sequences in progress revealed a Bacterial Artificial Chromosome (BAC) sequence (bK206c7) that contained matches to the *C. elegans* cosmid open reading frame, C54D2.5, and to the four human chromosome 22 ESTs, H55225, H55617, H55223, H55544. The C. elegans C54D2.5 cosmid sequence and the human EST sequences were then used to compare the translation of the bK206c7 BAC genomic sequence in all 6 reading frames. The analysis was performed using the graphical program Dotter (Eric Sohnhammer, NCBI). The analysis revealed a series of potential coding regions on one strand of the bK206c7 BAC sequence. These were subsequently translated in all 3 reading frames and the potential splice junctions identified. The translated sequence of this longer DNA fragment which is part of the human α₁ᵢ subunit gene is given by Seq. ID No. 17.

Using the sequence information from the four EST's, a full length gene can be recovered using any of several techniques. Polynucleotide probes having a sequence which corresponds to or hybridizes with the EST sequences or a distinctive portion thereof (for example oligonucleotide probes having a length of 18 to 100 nucleotides) can be used to probe a human cDNA library for identification of the full length DNA encoding the α₁ᵢ subunits. The process of identifying cDNAs of interest using defined probes is well known in the art and is, for example, described in International Patent Publication No. WO95/04144, which is incorporated herein by reference. This process generally involves screening bacterial hosts (e.g. *E. coli*) harboring the library plasmids or infected with recombinant lambda phage with labeled probes, e.g. radiolabeled with ³²P, and selection of colonies or phage which bind the labeled probe. Each selected colony or phase is grown up, and the plasmids are recovered. Human cDNAs are recovered from the plasmids by restriction digestion, or can be amplified, for example by PCR. The recovered cDNA can be sequenced, and the position of the calcium channel subunit-encoding region further refined, although neither process is not necessary to the further use of the cDNA to produce cell lines expressing the novel calcium channel subunits.

Longer portions of DNA-encoding the novel calcium channel subunits of the invention can also be recovered by PCR cloning techniques using primers corresponding to or based upon the EST sequences. Using this technique to identify relevant sequences within a human brain total RNA preparation confirmed that the novel α₁ᵢ calcium channel subunit is present in human brain. Subcloning of the 567 at PCR product and subsequent sequencing thereof showed that this product corresponds to the derived sequence form the bK206c7 BAC genomic sequence. The nucleotide sequence is given as SEQ ID No. 18. The same experiment was performed using a rat brain RNA preparation and resulted in recovery of a substantially identical PCR product. (SEQ ID. NO. 19). The protein encoded by the rat PCR product is 96% identical to the human PCR product.

These sequences, which presumably encode a partial subunit can be used as a basis for constructing full length human or rat α₁ᵢ clones. Briefly, the subcloned α₁ᵢ PCR product is radiolabeled by random hexamer priming according to standard methods (See, Sambrook, J., Fritsch, E.F. and Maniatis, T. (1989) Molecular Cloning, A Laboratory Manual. Cold Spring Harbor Press) and used to screen commercial human brain cDNA libraries (Stratagene, La Jolla, CA). The screening of cDNA libraries follows standard methods and includes such protocols as infecting bacteria with recombinant lambda phage, immobilizing lambda DNA to nitrocellulose filters and screening under medium hybridization stringency conditions with radiolabeled probe. cDNA clones homologous to the probe are identified by autoradiography. Positive clones are purified by sequential rounds of screening.

Following this protocol, most purified cDNA's are likely to be partial sequence clones due the nature of the cDNA library synthesis. Full length clones are constructed from cDNA's which overlap in DNA sequence. Restriction enzyme sites which overlap between cDNAs are used to ligate the individual cDNA's to generate a full-length cDNA. For subsequent heterologous expression, the full-length cDNA is subcloned directly into an appropriate vertebrate expression vector, such as pcDNA-3 (Invitrogen, San Diego, CA) in which expression of the cDNA is under the control of a promoter such as the CMV major intermediate early promoter/enhancer. Other suitable expression vectors include, for example, pMT2, pRC/CMV, pcDNA3.1 and pCEP4.

Once the full length cDNA is cloned into an expression vector, the vector is then transfected into a host cell for expression. Suitable host cells include *Xenopus* oocytes or mammalian cells such as human embryonic kidney cells as described in International Patent Publication No. WO 96/39512 which is incorporated herein by reference and Ltk cells as described in US Patent No. 5,386,025 which is incorporated herein by reference. Transfection into host cells may be accomplished by microinjection, lipofection, glycerol shock, electroporation calcium phosphate or particle-mediated gene transfer. The vector may also be transfected into host cells to provide coexpression of the novel α₁ subunits with a β and/or an α₂δ subunit.

The resulting cell lines expressing functional calcium channels including the novel α₁ subunits of the invention can be used test compounds for pharmacological activity with respect to these calcium channels. Thus, the cell lines are useful for screening compounds for pharmaceutical utility. Such screening can be carried out using several available methods for evaluation of the interaction, if any, between the test compound and the calcium channel. One such method involves the binding of radiolabeled agents that interact with the calcium channel and subsequent analysis of equilibrium binding measurements including but not limited to, on rates, off rates, K_{d} values and competitive binding by other molecules. Another such method involves the screening for the effects of compounds by electrophysiological assay whereby individual cells are impaled with a microelectrode and currents through the calcium channel are recorded before and after application of the compound of interest. Another method, high-throughput spectrophotometric assay, utilizes the loading the cell lines with a fluorescent dye sensitive to intracellular calcium concentration and subsequent examination of the effects of compounds on the ability of depolarization by potassium chloride or other means to alter intracellular calcium levels. Compounds to be tested as agonists or antagonists of the novel α₁ᵢ calcium channel subunits are combined with cells that are stably or transiently transformed with a DNA sequence encoding the α₁ᵢ calcium channel subunits of the invention and monitored using one of these techniques.

DNA fragments with sequences given by SEQ ID Nos. 13-19 may also be used for mapping the distribution of α₁₁ calcium channel subunits within a tissue sample. This method follows normal histological procedures using a nucleic acid probe, and generally involves the steps of exposing the tissue to a reagent comprising a directly or indirectly detectable label coupled to a selected DNA fragment, and detecting reagent that has bound to the tissue. Suitable labels include fluorescent labels, enzyme labels, chromophores and radio-labels.

### EXAMPLE 1

In order to isolate novel human calcium channel α₁ subunits using standard molecular cloning protocols, synthetic DNA probes are prepared, radiolabeled with ³²P and utilized to screen human cDNA libraries commercially available in lambda phage vectors (Stratagene, La Jolla, CA) based on the human DNA sequences for H55225, H53617, H55223, and H55544. DNA fragments with the sequence of sequence ID NOs 17 and 18 may also be used for this purpose. Positive phage are purified through several rounds of screening involving immobilizing the phage DNA on nitrocellulose filters, hybridizing with the radiolabeled probe, washing off of excess probe and then selection of clones by autoradiography. Clones identified by this approach are expected to be partial length clones due to the nature of cDNA library synthesis and several rounds of screening for each calcium channel type may be necessary to obtain full-length clones.

To characterize the clones, double stranded plasmid DNA is prepared from the identified clones and the sequences are determined using ³⁵S dATP, Sequenase and standard gel electrophoresis methods. Regions of similarity and regions of overlap are determined by comparison of each cDNA sequence.

Full-length clones are constructed by ligating overlapping cDNA fragments together at common restriction enzyme sites. The full-length clones are subsequently inserted into vectors suitable for expression in vertebrate cells (e.g. pMT2, pRC/CMV, pcDNA3.1, pCEP4. pREP7) by ligation into restriction sites in the vector polylinker region which is downstream of the promoter used to direct cDNA expression.

DNA encoding the novel calcium channels can be stably or transiently introduced into eukaryotic cells (e.g. human embryonic kidney, mouse L cells, chinese hamster ovary, etc) by any number of available standard methods. Stable transfection is achieved by growing the cells under conditions that promote growth of cells expressing a marker gene which is contained in the expression vector (e.g. dihydrofolate reductase, thymidine kinase, or the like). The heterologous DNA encoding the human calcium channel may be integrated into the genome or may be maintained as an episomal element.

Expression of the human calcium channel in transfected cells may monitored by any number of techniques, including Northern blot for RNA analysis. Southern blot for cDNA detection, electrophysiological assay for calcium channel function, the binding of radiolabeled agents thought to interact with the calcium channel, and fluorescent assay of dyes sensitive to intracellular calcium concentration.

### EXAMPLE 2

### Heterologous Expression of Human α₁ᵢ Calcium Channels in Cells

### A. Transient Transfection in Mammalian Cells

Host cells, such as human embryonic kidney cells, HEK 293 (ATCC# CRL 1573) are grown in standard DMEM medium supplemented with 2 mM glutamine and 10% fetal bovine serum. HEK 293 cells are transfected by a standard calcium-phosphate-DNA coprecipitation method using the full-lenngth human α₁ᵢ calcium channel cDNA in a vertebrate expression vector (for example see Current protocols in Molecular Biology). The human α₁ᵢ calcium channel cDNA may be transfected alone or in combination with other cloned subunits for mammalian calcium channels, such as α2δ and β subunits, and also with clones for marker proteins such the jellyfish green fluorescent protein.
Electrophysiological Recording: After an incubation period of from 24 to 72 hrs the culture medium is removed and replaced with external recording solution (see below). Whole cell patch clamp experiments are performed using an Axopatch 200B amplifier (Axon Instruments, Burlingame, CA) linked to an IBM compatible personal computer equipped with pCLAMP software. Microelectrodes are filled with 3 M CsCl and have typical resistances from 0.5 to 2.5 M_. The external recording solution is 20 mM BaCl₂, 1 mM MgCl₂, 10 mM HEPES, 40 mM TEACl, 10 mM Glucose, 65 mM CsCl, (pH 7.2). The internal pipette solution is 105 mM CsCl, 25 mM TEACl, 1 mM CaCl₂, 11 mM EGTA, 10 mM HEPES (pH 7.2). Currents are typically elicited from a holding potential of -100 mV to various test potentials. Data are filtered at 1 kHz and recorded directly on the harddrive of a personal computer. Leak subtraction is carried out on-line using a standard P/5 protocol. Currents are analyzed using pCLAMP versions 5.5 and 6.0. Macroscopic current-voltage relations are fitted with the equation 1 = {1/(1+exp(-(Vₘ-Vₕ)/S)} x G - (Vₘ-Eᵣₑᵥ), where Vₘ is the test potential. Vₕ is the voltage at which half of the channels are activated, and S reflects the steepness of the activation curve and is an indication of the effective gating charge movement. Inactivation curves are normalized to 1 and fitted with 1 = (1/1 + exp ((Vₘ-Vₕ)/S) with Vₘ being the holding potential. Single channel recordings are performed in the cell-attached mode with the following pipette solution (in mM): 100 BaCl₂, 10 HEPES, pH 7.4 and bath solution: 100 KCl, 10 EGTA. 2 MgCl₂, 10 HEPES, pH 7.4.

### B. Transient Transfection in Xenopus Oocytes

Stage V and VI Xenopus oocytes are prepared as described by Dascal et al (1986), Expression and modulation of voltage-gated calcium channels after RNA injection into Xenopus oocytes. Science 231:1147-1150. After enzymatic dissociation with collagenase, oocytes nuclei are microinjected with the human α₁ᵢ calcium channel cDNA expression vector construct (approximately 10 ng DNA per nucleus) using a Drummond nanoject apparatus. The human α₁ᵢ calcium channel may be injected alone, or in combination with other mammalian calcium channel subunit cDNAs, such as the α2-δ and β1b subunits. After incubation from 48 to 96 hrs macroscopic currents are recorded using a standard two microelectrode voltage-clamp (Axoclamp 2A, Axon Instruments, Burlingame, CA) in a bathing medium containing (in mM): 40 Ba(OH)_{2,}, 25 TEA-OH, 25 NaOH, 2 CsOH, 5 HEPES (pH titrated to 7.3 with methan-sulfonic acid). Pipettes of typical resistance ranging from 0.5 to 1.5 m_ are filled with 2.8M CsCl, 0.2M CsOH, 10mM HEPES, 10mM BAPTA free acid. Endogenous Ca (and Ba) -activated Cl currents are suppressed by systematically injecting 10-30 nl of a solution containing 100mM BAPTA-free acid, 10mM HEPES (pH titrated to 7.2 with CsOH) using a third pipette connected to a pneumatic injector. Leak currents and capacitive transients are subtracted using a standard P/5 procedure.

### EXAMPLE 3

### Construction of Stable Cell Lines Expressing Human α₁ᵢ Calcium Channels

Mammalian cell lines stably expressing human α₁ᵢ calcium channels are constructed by transfecting the α₁ᵢ calcium channel cDNA into mammalian cells such as HEK 293 and selecting for antibiotic resistance encoded for by an expression vector. Briefly, the full-length human α₁ᵢ calcium channel cDNA subcloned into a vertebrate expression vector with a selectable marker, such as the pcDNA3 (InvitroGen, San Diego, CA), is transfected into HEK 293 cells by calcium phosphate coprecipitation or lipofection or electroporation or other method according to well known procedures (Methods in Enzymology, Volume 185, Gene Expression Technology (1990) Edited by Goeddel, D.V.). The human α₁ᵢ calcium channel may be transfected alone, or in combination with other mammalian calcium channel subunit cDNAs, such as the α2-δ and β1b subunits, either in a similar expression vector or other type of vector using different selectable markers. After incubation for 2 days in nonselective conditions, the medium is supplemented with Geneticin (G418) at a concentration of between 600 to 800 ug/ml. After 3 to 4 weeks in this medium, cells which are resistant to G418 are visible and can be cloned as isolated colonies using standard cloning rings. After growing up each isolated colony to confluency to establish cell lines, the expression of human α₁ᵢ calcium channels can be determined at with standard gene expression methods such as Northern blotting, RNase protection and reverse-transcriptase PCR.

The functional detection of human α₁ᵢ calcium channels in stably transfected cells can be examined electrophysiologically, such as by whole patch clamp or single channel analysis (see above). Other means of detecting functional calcium channels include the use of radiolabeled ⁴⁵Ca uptake, fluorescence spectroscopy using calcium sensitive dyes such as FURA,-2, and the binding or displacement of radiolabeled ligands that interact with the calcium channel.

## Claims

1. An recombinant DNA molecule which comprises an expression system for the production of a calcium ion channel α₁ᵢ subunit protein which expression system comprises
a nucleotide sequence encoding a functional calcium channel α₁ᵢ subunit or the complement to said encoding nucleotide sequence, wherein said encoding nucleotide sequence comprises a nucleotide sequence encoding the amino acid sequence encoded by SEQ. ID. NO: 17.

2. The DNA molecule of claim 1 wherein said encoding nucleotide sequence is that set forth in SEQ. ID. NO: 17.

3. Recombinant host eukaryotic cells which are modified to contain the DNA molecule of claim 1 or 2.

4. A method to produce a functional calcium ion channel α₁ᵢ subunit protein which method comprises culturing the cells of claim 3 under conditions wherein said expression system produces said protein.

5. A method to prepare cells which produce a functional calcium ion channel α₁ᵢ subunit protein which method comprises introducing into said cells the DNA molecule of any of claims 1-2.

6. An isolated recombinantly produced protein functional as an activated calcium channel α₁ᵢ subunit which protein comprises an amino acid sequence encoded by SEQ. ID. NO: 17.

7. Recombinant eukaryotic cells that display the protein of claim 6.

8. A method to identify a compound that acts as an agonist or an antagonist for a calcium ion channel α₁ᵢ subunit, which method comprises contacting the cells of claim 7 with a compound to be tested and evaluating the interaction, if any, between the compound to be tested and the displayed protein,
wherein a compound that activates the flow of calcium ions is identified as an agonist, and
a compound that blocks the flow of calcium ion is identified as an antagonist.

9. A method to map the distribution of calcium ion channel α₁ᵢ subunits in a tissue sample, which method comprises
exposing the tissue to a reagent that comprises antibodies to the protein of claim 6, or with a nucleotide sequence that hybridizes to SEQ. ID. NO: 17 under conditions of moderate stringency, and
detecting the location of binding of said reagent.

## Patentansprüche

1. Rekombinantes DNA-Molekül, umfassend ein Expressionssystem zur Herstellung eines Kalziumionenkanal α₁ᵢ-Untereinheitproteins, wobei das Expressionssystem eine Nukleotidsequenz umfasst, die eine funktionelle Kalziumkanal α₁ᵢ-Untereinheit oder das Komplement zu der besagten kodierenden Nukleotidsequenz kodiert, wobei die besagte kodierende Nukleotidsequenz eine Nukleotidsequenz umfasst, die für die Aminosäuresequenz kodiert, die von SEQ. ID. NO: 17 kodiert wird.

2. DNA-Molekül nach Anspruch 1, worin die besagte kodierende Nukleotidsequenz jene ist, die in SEQ. ID. NO: 17 dargelegt ist.

3. Rekombinante eukaryotische Wirtszellen, welche modifiziert sind, um das DNA-Molekül nach Anspruch 1 oder 2 zu enthalten.

4. Verfahren zur Herstellung eines funktionellen Kalziumionenkanal α₁ᵢ-Untereinheitproteins, wobei das Verfahren die Kultivierung der Zellen nach Anspruch 3 unter Bedingungen umfasst, unter welchen das besagte Expressionssystem das besagte Protein herstellt.

5. Verfahren zur Herstellung von Zellen, die ein funktionelles Kalziumionenkanal α₁ᵢ-Untereinheitprotein herstellen, wobei das Verfahren die Einführung des DNA-Moleküls nach einem der Ansprüche 1-2 in die besagten Zellen umfasst.

6. Isoliertes, rekombinant hergestelltes Protein, das als eine aktivierte Kalziumkanal α₁ᵢ-Untereinheit funktional ist, wobei das Protein eine Aminosäuresequenz umfasst, die von SEQ. ID. NO: 17 kodiert wird.

7. Rekombinante eukaryotische Zellen, die das Protein nach Anspruch 6 präsentieren.

8. Verfahren um eine Verbindung zu identifizieren, die als ein Agonist oder Antagonist für eine Kalziumionenkanal α₁ᵢ-Untereinheit wirkt, wobei das Verfahren das in Kontakt bringen der Zellen nach Anspruch 7 mit einer zu testenden Verbindung und die Evaluierung der Interaktion, wenn überhaupt, zwischen der zu testenden Verbindung und dem präsentierten Protein umfasst, wobei eine Verbindung, die den Fluss von Kalziumionen aktiviert, als ein Agonist und eine Verbindung, die den Fluss von Kalziumionen blockiert, als ein Antagonist identifiziert wird.

9. Verfahren, um die Verteilung von Kalziumionenkanal α₁ᵢ-Untereinheiten in einer Gewebeprobe aufzuzeichnen, wobei das Verfahren die Exposition des Gewebes zu einem Reagenz umfasst, das Antikörper zu dem Protein nach Anspruch 6 umfasst, oder zu einer Nukleotidsequenz, die an SEQ. ID. NO: 17 unter Bedingungen moderater Stringenz hybridisiert, und die Detektion der Lokalisation der Bindung des besagten Reagenzes.

## Revendications

1. Molécule d'ADN recombiné comprenant un système d'expression pour la production d'une sous-unité protéique α₁ᵢ de canal d'ion calcium, lequel système d'expression comprend
une séquence de nucléotides codant pour une sous-unité α₁ᵢ de canal calcique fonctionnelle ou le complément de ladite séquence de nucléotides codante, dans lequel ladite séquence de nucléotides codante comprend une séquence de nucléotides codant la séquence d'acides aminés codée par SEQ ID n° : 17.

2. Molécule d'ADN selon la revendication 1, dans laquelle ladite séquence de nucléotides codante est celle qui est désignée par SEQ ID n° : 17.

3. Cellules hôtes eucaryotes recombinées modifiées pour contenir la molécule d'ADN selon la revendication 1 ou 2.

4. Procédé de production de sous-unité protéique α₁ᵢ de canal d'ion calcium fonctionnelle, lequel procédé comprend la culture des cellules selon la revendication 3 dans des conditions dans lesquelles ledit système d'expression produit ladite protéine.

5. Procédé de préparation de cellules produisant une sous-unité protéique α₁ᵢ de canal d'ion calcium fonctionnelle, lequel procédé comprend l'introduction dans lesdites cellules de la molécule d'ADN selon l'une quelconque des revendications 1 ou 2.

6. Protéine isolée produite par recombinaison et fonctionnelle en tant que sous-unité α₁ᵢ de canal calcique activée, laquelle protéine comprend la séquence d'acides aminés codée par SEQ ID n° : 17.

7. Cellules eucaryotes recombinées présentant la protéine selon la revendication 6.

8. Procédé d'identification d'un composé jouant le rôle d'agoniste ou d'antagoniste d'une sous-unité α₁ᵢ de canal d'ion calcium, lequel procédé comprend la mise en contact des cellules selon la revendication 7 avec un composé à tester et l'évaluation de l'interaction, le cas échéant, entre le composé à tester et la protéine présentée,
dans lequel un composé activant la circulation des ions calcium est identifié en tant qu'agoniste et
un composé qui bloque la circulation des ions calcium est identifié en tant qu'antagoniste.

9. Procédé pour cartographier la répartition des sous-unités α₁ᵢ de canal d'ion calcium dans un échantillon de tissu, lequel procédé comprend l'exposition du tissu à un réactif comprenant des anticorps dirigés contre la protéine selon la revendication 6 ou avec une séquence de nucléotides s'hybridant à SEQ ID n° : 17 dans des conditions de stringence modérées et
la détection du site de liaison dudit réactif.


### SEQUENCE LISTING

(1) GENERAL INFORMATION:
   (i) APPLICANT: Snutch, Terry P. Baillie, David L.
   (ii) TITLE OF INVENTION: Novel Human Calcium Channels and Related Probes, Cell Lines and Methods
   (iii) NUMBER OF SEQUENCES: 19
   (iv) CORRESPONDENCE ADDRESS:
   (A) ADDRESSEE:
   (B) STREET:
   (C) CITY:
   (D) STATE:
   (E) COUNTRY:
   (F) ZIP:
   (v) COMPUTER READABLE FORM:
   (A) MEDIUM TYPE: Diskette, 3.50 inch, 1.44 Kb storage
   (B) COMPUTER: IBM Compatible
   (C) OPERATING SYSTEM: MS DOS 6.0
   (D) SOFTWARE: WordPerfect
   (vi) CURRENT APPLICATION DATA:
   (A) APPLICATION NUMBER:
   (B) FILING DATE:
   (C) CLASSIFICATION:
   (viii) ATTORNEY/AGENT INFORMATION:
   (A) NAME: Larson, Marina T.
   (B) REGISTRATION NUMBER: 32038
   (C) REFERENCE/DOCKET NUMBER: NMED.P-001-US
   (ix) TELECOMMUNICATION INFORMATION:
   (A) TELEPHONE: (914) 245-3252
   (B) TELEFAX: (914) 962-4330
(2) INFORMATION FOR SEQ ID NO: 1:
   (i) SEQUENCE CHARACTERISTICS:
   (A) LENGTH: 24
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO:1:
   GTCAAAACTC AGGCCTTCTA CTGG 24
(2) INFORMATION FOR SEQ ID NO: 2:
   (i) SEQUENCE CHARACTERISTICS:
   (A) LENGTH: 24
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 2:
   AACGTGTTCT TGGCTATCGC GGTG 24
(2) INFORMATION FOR SEQ ID NO:3:
   (i) SEQUENCE CHARACTERISTICS:
   (A) LENGTH: 24
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 3:
   GTGAAAGCAC AGAGCTTCTA CTGG 24
(2) INFORMATION FOR SEQ ID NO: 4:
   (i) SEQUENCE CHARACTERISTICS:
   (A) LENGTH: 24
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 4:
   AACGTTTTCT TGGCCATTGC TGTG 24
(2) INFORMATION FOR SEQ ID NO: 5:
   (i) SEQUENCE CHARACTERISTICS:
   (A) LENGTH: 24
   (B) TYPE: nucleic acid
   (C) STRANDEDNESS: single
   (D) TOPOLOGY: linear
   (ii)MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 5:
(2) INFORMATION FOR SEQ ID NO: 6:
   (i) SEQUENCE CHARACTERISTICS:
   (A) LENGTH: 24
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 6:
   AATGTGTTCT TGGCCATTGC GGTG 24
(2) INFORMATION FOR SEQ ID NO: 7:
   (i) SEQUENCE CHARACTERISTICS:
   (A) LENGTH: 24
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 7:
   GTGAAGTCTG TCACGTTTTA CTGG 24
(2) INFORMATION FOR SEQ ID NO: 8:
   (i) SEQUENCE CHARACTERISTICS:
   (A) LENGTH: 24
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 8:
   AAGCTCTTCT TGGCCATTGC TGTA 24
(2) INFORMATION FOR SEQ ID NO: 9:
   (i) SEQUENCE CHARACTERISTICS:
   (A) LENGTH: 24
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 9:
   GTCAAGTCGC AAGTGTTCTA CTGG 24
(2) INFORMATION FOR SEQ ID NO: 10:
   (i) SEQUENCE CHARACTERISTICS:
   (A) LENGTH: 24
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 10:
   AATGTATTCT TGGCTATCGC TGTG 24
(2) INFORMATION FOR SEQ ID NO: 11:
   (i) SEQUENCE CHARACTERISTICS:
   (A) LENGTH: 21
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 11:
   ATCTAYGCYR TSATYGGSAT G 21
(2) INFORMATION FOR SEQ ID NO: 12:
   (i) SEQUENCE CHARACTERISTICS:
   (A) LENGTH: 20
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: oligonucleotide probe for locating calcium channel genes
   (xi) SEQUENCE DESCRIPTION: SEQ ID NO: 12:
   ATGGACAAYT TYGASTAYTC 20
(2) INFORMATION FOR SEQ ID NO: 13:
   (i) SEQUENCE CHARACTERISTICS:
   (A) LENGTH: 168
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: human
   (ix) FEATURE: expressed sequence tag H55225
   (xi) SEQUENCE DESCRIPTION: SEQ ID NO: 13:
   GTGATCACTC TGGAAGGCTG GGTGGAGATC ATGTACTACG TGATGGATGC TCACTCCTTC  60
   TACAACTTCA TCTACTTCAT CCTGCTTATC ATACCCCTCT TGCCTTGCAC CCCATATGGT 120
   CTTCCCAGAG TGAGCTCATC CACCTCGTCA TGCCTGACTC GACGTTCA              168
(2) INFORMATION FOR SEQ ID NO: 14:
   (i) SEQUENCE CHARACTERISTICS:
   (A) LENGTH: 98
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: human
   (ix) FEATURE: expressed sequence tag H55617
   (xi) SEQUENCE DESCRIPTION: SEQ ID NO: 14:
   GATGGTCGAG TACTCCCTGG ACCTTCAGAA CATCAACCTG TCAGCCATCC GCACCGTGCG  60
   CGTCCTGAGG CCCCTCAAAG CCATCAACCG CGTGCCCA                          98
(2) INFORMATION FOR SEQ ID NO: 15:
   (i) SEQUENCE CHARACTERISTICS:
   (A) LENGTH: 94
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: human
   (ix) FEATURE: expressed sequence tag H55223
   (xi) SEQUENCE DESCRIPTION: SEQ ID NO: 15:
   CATGCTGGTG ATCCTGCTGA ACTGCGTGAC ACTTGGCATG TACCAGCCGT GCGACGACAT    60
   GGACTGCCTG TCCGACCGCT GCAAGATCCT GCAG                                94
(2) INFORMATION FOR SEQ ID NO: 16:
   (i) SEQUENCE CHARACTERISTICS:
   (A) LENGTH: 123
   (B) TYPE: nucleic acid
   (C) STRANDEDNESS: single
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: human
   (ix) FEATURE: expressed sequence tag H55544
   (xi) SEQUENCE DESCRIPTION: SEQ ID NO: 16:
   GTATCTCTGG TTACTTTAGT AGCCAACACT CTTGGCTACT CAGACCTTGG TCCCATTAAA    60
   TCCCTGCGAA CCTTGAGAGC ACTAAGACCT CTAAGAGCTT TGTCTAGATT TGAAGGAATG   120
   AGG                                                                 123
(2) INFORMATION FOR SEQ ID NO: 17:
   (i) SEQUENCE CHARACTERISTICS:
   (A) LENGTH: 5562
   (B) TYPE: nucleic acid
   (C) STRANDEDNESS: double
   (D) TOPOLOGY: linear
   (ii) MOLECULE TYPE: cDNA
   (iii) HYPOTHETICAL: no
   (iv) ANTl-SENSE: no
   (vi) ORIGINAL SOURCE:
   (A) ORGANISM: human
   (ix) FEATURE: human alpha-I partial sequence from BAC bK206c7
   (xi) SEQUENCE DESCRIPTION: SEQ ID NO: 17:
(2) INFORMATION FOR SEQ ID NO: 18:
   (i) SEQUENCE CHARACTERISTICS:
   (A) LENGTH: 567
   (B) TYPE: nucleic acid
   (C) STRANDEDNESS: double
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: human
   (ix) FEATURE: human alpha-I partial sequence
   (xi) SEQUENCE DESCRIPTION: SEQ ID NO: 18:
   ATG CGC ATC CTG GTG AAC CTG CTC CTG GAC ACA CTG CCC ATG CTG GGG AAT GTC 54
   Met Arg Ile Leu Val Asn Leu Leu Leu Asp Thr Leu Pro Met Leu Gly Asn Val
   CTG CTG CTC TGC TTC TTT GTC TTC TTC ACC TTT GGC ATC ATA GGT GTG CAG CTC 108
   Leu Leu Leu Cys Phe Phe Val Phe Phe Thr Phe Gly Ile Ile Gly Val Gln Leu
   TGG GCG GGC CTG CTG CGT AAC CGC TGC TTC CTG GAG GAG AAC TTC ACC ATA CAA 162
   Trp Ala Gly Leu Leu Arg Asn Arg Cys Phe Leu Glu Glu Asn Phe Thr Ile Gln
   GGG GAC GTG GCC TTG CCC CCA TAC TAC CAG CCG GAG GAG GAT GAT GAG ATG CCC 216
   Gly Asp Val Ala Leu Pro Pro Tyr Tyr Gln Pro Glu Glu Asp Asp Glu Met Pro
   TTC ATC TGC TCC CTG TCG GGC GAC AAT GGG ATA ATG GGC TGC CAT GAG ATC CCC 270
   Phe Ile Cys Ser Leu Ser Gly Asp Asn Gly Ile Met Gly Cys His Glu Ile Pro
   CCG CTC AAG GAG CAG GGC CGT GAG TGC TGC CTG TCC AAG GAC GAC GTC TAC GAC 324
   Pro Leu Lys Glu Gln Gly Arg Glu Cys Cys Leu Ser Lys Asp Asp Val Tyr Asp
   TTT GGG GCG GGG CGC CAG GAC CTC AAT GCC AGC GGC CTC TGT GTC AAC TGG AAC 378
   Phe Gly Ala Gly Arg Gln Asp Leu Asn Ala Ser Gly Leu Cys Val Asn Trp Asn
   CGT TAC TAC AAT GTG TGC CGC ACG GGC AGC GCC AAC CCC CAC AAG GGT GCC ATC 432
   Arg Tyr Tyr Asn Val Cys Arg Thr Gly Ser Ala Asn Pro His Lys Gly Ala Ile
   AGC TTT GAC AAC ATC GGT TAT GCT TGG ATT GTC ATC TTC CAG GTG ATC ACT CTG 486
   Ser Phe Asp Asn Ile Gly Tyr Ala Trp Ile Val Ile Phe Gln Val Ile Thr Leu
   GAA GGC TGG GTG GCG ATC ATG TAC TAC GTG ATG GAT GCT CTC TCC TTC TAC AAC 540
   Glu Gly Trp Val Ala Ile Met Tyr Tyr Val Met Asp Ala Leu Ser Phe Tyr Asn
   TTC GTC TAC TTC ATC CTG CTT ATC ATA 567
   Phe Val Tyr Phe Ile Leu Leu Ile Ile
(2) INFORMATION FOR SEQ ID NO: 19 :
   (i) SEQUENCE CHARACTERISTICS:
   (A) LENGTH: 567
   (B) TYPE: nucleic acid
   (C) STRANDEDNESS: double
   (D) TOPOLOGY: linear
   (ii) MOLECULE TYPE: other nucleic acid
   (iii) HYPOTHETICAL: no
   (iv) ANTI-SENSE: no
   (vi) ORIGINAL SOURCE:
   (A) ORGANISM: rat
   (ix) FEATURE: rat alpha-I partial sequence
   (xi) SEQUENCE DESCRIPTION: SEQ ID NO: 19: